# DEMANDE DE BREVET EUROPEEN

(11) **EP 4 628 068 A1**
(43) Date de publication de la demande: **08.10.2025**
(21) Numéro de dépôt: 25166392.8
(22) Date de dépôt: 26.03.2025
(51) Int. Cl.: A61K 8/92, A61Q 5/12

(54) **COMPOSITIONS DE SOINS POUR CHEVEUX TEXTURÉS, NOTAMMENT LES CHEVEUX AFRO-TEXTURÉS**

(30) Priorité: 26.03.2024 FR 2403041
(71) Demandeur: Morphoeus, 97300 Cayenne (GY)
(72) Inventeur: BLIN, Sandrina, 97300 CAYENNE (GY); KNIPPER, Magali, 97300 CAYENNE (GY)
(74) Mandataire: Roman, Alexis

(57) **Abrégé**

L'invention concerne une association d'huiles végétales, destinée aux soins des cheveux texturés, notamment des cheveux afro-texturés, ladite association étant constituée d'au moins 20% p/p_{association} d'huile d'Abyssine, d'au moins 20% p/passociation d'huile de baobab, et d'au moins 5% p/p_{association} d'huile de lin et/ou d'huile de carthame.

## Description

### Domaine technique.

La présente invention concerne une composition de soins pour cheveux texturés, notamment les cheveux afro-texturés, comprenant une association spécifique d'huile de Baobab; d'huile d'Abyssinie; et d'huile de lin et/ou d'huile de carthame.

L'invention vise également l'association d'huiles végétales mise en œuvre dans ladite composition de soins.

L'invention concerne aussi l'utilisation de ladite association ou de ladite composition cosmétique, pour le traitement et le soin desdits cheveux texturés.

La présente invention se situe dans le domaine des soins capillaires, et notamment des compositions et procédés conçus pour hydrater, réparer, définir et lustrer les cheveux textures.

### État de la technique.

Les cheveux, éléments essentiels de notre apparence et de notre identité, sont composés de structures complexes qui demandent des soins spécifiques pour les maintenir en bonne santé et en bon état. Le cheveu est constitué de la tige capillaire, une structure qui comprend trois composants principaux : la médulla (couche interne), le cortex (couche médiane) et la cuticule (couche externe). Cette tige capillaire, qui pousse à partir du follicule pileux, est le siège de nombreuses propriétés esthétiques et fonctionnelles des cheveux, notamment leur force, leur élasticité, leur couleur et leur résistance aux agressions extérieures.

Parmi les éléments essentiels qui composent le cheveu, on trouve des lipides internes tels que les céramides, les acides gras et les stérols, ainsi que la kératine qui représente plus de 90% de sa composition totale. Les lipides, présents à l'intérieur du cortex et de la cuticule, jouent un rôle fondamental dans le maintien de l'intégrité et de la santé du cheveu, contribuant ainsi à sa structure, à sa résistance, et à sa capacité à retenir l'humidité nécessaire. La kératine, une protéine fibreuse composée de multiples acides aminés, confère aux cheveux leur structure et leur résistance. De plus, elle a la remarquable capacité d'absorber jusqu'à 40 % de son poids en eau, permettant aux cheveux de maintenir leur teneur en humidité et d'éviter le dessèchement.

Il est important de noter que les cheveux présentent une grande diversité de textures, allant des cheveux lisses aux cheveux crépus. Chaque type de cheveux peut avoir des besoins spécifiques en matière de soins et de traitements, particulièrement les cheveux texturés - un terme souvent utilisé pour décrire les types de cheveux des catégories 2, 3 et 4 selon la classification d'André Walker.

Les cheveux texturés sont connus pour leur variété et leur caractère unique. Ils comprennent des textures capillaires ondulées, bouclées et crépues, et sont fréquemment observés chez des individus d'origines ethniques variées, tels que les Africains, les Afro-Américains, les Afro-Caribéens, les Latino-Américains et d'autres groupes ethniques du monde entier.

Les cheveux de type 2 présentent une texture ondulée, tandis que les cheveux de type 3 ont des boucles qui sont définies. Les cheveux de type 4, eux ont des boucles plus re-serrées, avec des rayons de courbure d'intensité variables, et comprennent la texture de cheveux crépue - aussi appelée afro-texturée - et la texture des cheveux frisés. Il est important de noter que ces personnes peuvent avoir (et ont souvent) plusieurs textures de cheveux présentes sur leur cuir chevelu.

Les cheveux texturés, véritables symboles de beauté naturelle, présentent des caractéristiques uniques qui les distinguent. Ces cheveux, avec leurs structures en spirales très variées, sont uniques pour leur diversité et leur aspect volumineux. Ils peuvent aussi représenter pour une certaine partie de la population un certain nombre de défis en termes de soins quotidiens et de coiffure, car ils nécessitent des techniques, des connaissances et des rituels de beauté particuliers qui sont souvent méconnus et restent encore à explorer. Cette configuration en spirale spécifique a de nombreux atouts, notamment en termes de créativité capillaire, mais elle complique aussi la répartition uniforme des lipides internes, naturellement produits par le cuir chevelu, le long de la tige capillaire. En conséquence, les personnes aux cheveux texturés sont fréquemment confrontées à une perte d'hydratation, de brillance et de souplesse, augmentant ainsi le risque de casse. De plus, leur entretien quotidien peut s'avérer ardu, avec des difficultés de démêlage et de coiffage, ce qui peut engendrer frustration et découragement. En outre, ces cheveux sont particulièrement vulnérables aux agressions environnementales, telles que l'exposition au soleil, à l'eau chlorée ou salée, et aux traitements capillaires fréquents, exacerbant ainsi les défis liés à leur soin.

Les produits de soin capillaire disponibles sur le marché sont majoritairement conçus pour les cheveux lisses ou légèrement ondulés, qui ont des besoins spécifiques. Ces produits sont formulés avec des ingrédients et des technologies qui répondent à ces besoins. Cependant, ils ne sont pas toujours adaptés aux cheveux texturés, qui ont des besoins différents. Par exemple, les produits de soin capillaire contenant des silicones peuvent laisser un aspect gras et collant sur les cheveux texturés. Les silicones sont des substances synthétiques qui forment un film protecteur sur les cheveux. Ce film peut aider à protéger les cheveux des dommages, mais il peut également rendre les cheveux lourds et difficiles à coiffer. D'autres ingrédients couramment utilisés dans les produits de soin capillaire, tels que les sulfates et les parabènes, peuvent également causer des problèmes pour les cheveux et le cuir chevelu. Les sulfates sont des tensioactifs qui aident à nettoyer les cheveux. Cependant, ils peuvent être agressifs pour les cheveux texturés, qui sont déjà plus secs et plus fragiles que les cheveux lisses. Ils peuvent provoquer des irritations, des pellicules et de la sécheresse. Les parabènes sont des conservateurs synthétiques couramment utilisés dans les produits capillaires. Cependant, ils peuvent agir comme des perturbateurs endocriniens et/ou provoquer des réactions allergiques et des irritations. Ils peuvent également décolorer les cheveux et les rendre ternes.

En conséquence, il existe donc un réel besoin pour des produits capillaires qui répondent aux besoins spécifiques des cheveux texturés. Ces produits doivent être capables de maintenir l'hydratation des cheveux, faciliter le démêlage et le coiffage, fournir une plus grande brillance et une meilleure (re)définition des boucles tout en procurant un effet soyeux. Il existe également un besoin pour des produits capillaires qui soient exempts d'ingrédients chimiques agressifs et peu coûteux. Un objet de l'invention est de satisfaire, au moins partiellement, ces besoins.

### Présentation de l'invention.

Il est du mérite de la Demanderesse d'avoir développé une association d'huiles végétales particulièrement efficace, spécifiquement conçue pour répondre aux besoins spécifiques des cheveux texturés, notamment des cheveux afro-texturés, en particulier des cheveux de type 3 à 4 selon la classification d'André Walker.

Cette association d'huiles agit en synergie pour traiter efficacement les problématiques couramment rencontrées avec ce type de chevelure, telles que la sécheresse, la difficulté de démêlage et la perte de définition des boucles.

L'association développée selon l'invention est constituée d'au moins 20 % en poids d'huile d'Abyssinie, d'au moins 20 % en poids d'huile de baobab, et d'au moins 5 % en poids d'huile de lin et/ou d'huile de carthame. Les huiles de l'association ou de la composition de soins capillaire développée par la Demanderesse sont des ingrédients naturels riches en acides gras essentiels qui aident à hydrater, à nourrir et à protéger les cheveux texturés, notamment en apportant ou améliorant la facilité de peignage et de démêlage, la définition des boucles, le toucher et la brillance des cheveux texturés. De telles huiles ou acides gras essentiels contribuent également à empêcher ou limiter la perte d'humidité des cheveux, la formation d'extrémités fendues ou fourches, la perte d'élasticité et/ou la casse des cheveux. Par ailleurs, grâce à leurs propriétés siccatives, ces huiles favorisent la polymérisation des acides gras polyinsaturés à la surface des cheveux, conduisant à la formation d'un film protecteur non gras, qui améliore la rétention d'hydratation et offre une protection supplémentaire contre les agressions extérieures. Elles ne provoquent aucune réaction d'intolérance ou d'irritation cutanée. Enfin, elles sont rapidement absorbées par les cheveux sans laisser de film gras.

L'huile de lin (nom **INCI :** *Linum usitatissimum Seed Oil*) est une huile végétale obtenue à partir des graines de la plante de lin (*Linum usitatissimum*)*.* Elle se distingue par sa richesse en acides gras essentiels, notamment l'acide alpha-linolénique (C18:3, n-3), un acide gras polyinsaturé de la famille des oméga-3, ainsi que l'acide linoléique (C18:2, n-6), un acide gras polyinsaturé appartenant à la famille des oméga-6. Au fil des siècles, l'huile de lin a trouvé de nombreuses applications dans divers secteurs tels que l'alimentation, les cosmétiques, la peinture, et bien d'autres encore.

L'huile de carthame (nom **INCI :** Carthamus tinctorius Seed Oil) est une huile végétale extraite des graines d'une plante herbacée oléagineuse appelée carthame des teinturiers, originaire d'Afrique du Nord et d'Asie occidentale. Cette huile est réputée pour sa richesse en acide linoléique (65 à 85%). Elle a de nombreuses applications dans l'industrie alimentaire, cosmétique et pharmaceutique.

L'huile de baobab (nom **INCI :** Adansonia Digitata Seed Oil) est une huile végétale extraite des graines contenues dans les fruits du baobab, un arbre emblématique qui pousse principalement en Afrique sub-saharienne. L'huile de baobab est réputée pour sa richesse en acides gras essentiels, notamment en acide oléique (composant prédominant), un acide gras monoinsaturé de la famille des oméga-9. Cette huile trouve une multitude d'applications, notamment dans l'industrie cosmétique et alimentaire, en raison de ses bienfaits pour la peau et les cheveux, ainsi que de sa teneur en nutriments bénéfiques pour la santé.

L'huile d'Abyssinie (nom **INCI :** Crambe Abyssinica Seed Oil) est une huile végétale extraite des graines de Crambe abyssinica, une plante oléagineuse annuelle appartenant à la famille des Brassicaceae. Cette plante est originaire des contreforts abyssiniens de l'Éthiopie ainsi que des plaines d'Afrique du Nord. L'huile d'Abyssinie se distingue par sa richesse en acides gras insaturés, principalement en acide érucique (environ 50 %), qui est un acide gras monoinsaturé de la famille des oméga-9. L'huile d'Abyssinie est utilisée dans de nombreuses applications, notamment dans l'alimentation, les cosmétiques et l'industrie.

Cependant, à la connaissance de la Demanderesse, aucune association d'huiles végétales destinée aux soins des cheveux texturés ne comprend, à l'heure actuelle, une combinaison définie par au moins 20 % en poids d'huile d'Abyssinie ; au moins 20 % en poids d'huile de baobab ; et au moins 5 % en poids d'huile de lin et/ou d'huile de carthame.

Ainsi, selon un aspect de l'invention, il est proposé une association d'huiles végétales, remarquable en ce qu'elle est constituée d'huile d'Abyssinie, d'huile de baobab, et d'huile de lin et/ou d'huile de carthame, dans les proportions précitées, et spécifiquement destinée aux soins des cheveux texturés, notamment des cheveux afro-texturés.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaire.

Avantageusement, l'association selon la présente invention est capable de former un film protecteur à la surface des cheveux par polymérisation de l'acide linoléique (C18:2, n-6) et/ou de l'acide linolénique (C18:3, n-3) qu'elle contient, lorsqu'elle est appliquée sur les cheveux.

La polymérisation de l'acide linoléique (C18:2, n-6) et/ou de l'acide linolénique (C18:3, n-3) peut être activée par l'exposition à l'oxygène de l'air ambiant, à un rayonnement ultraviolet et/ou à la chaleur, notamment à une température comprise entre 25°C et 100°C.

Selon un aspect particulier de la présente invention, l'huile d'Abyssinie est présente dans l'association d'huiles végétales en une teneur de 25 à 65% p/p_{association}.

Selon un aspect particulier de la présente invention, l'huile de lin et/ou l'huile de carthame est présente dans l'association d'huiles végétales en une teneur de 5 à 30% p/p_{association}.

Selon un aspect particulier de la présente invention, l'huile de baobab est présente dans l'association d'huiles végétales en une teneur de 25 à 65% p/p_{association}.

De préférence, l'huile de baobab est présente à une teneur de 30 à 60% p/p_{association} ; l'huile d'Abyssinie est présente à une teneur de 30 à 60% p/p_{association} ; l'huile de lin et/ou l'huile de carthame est présente à une teneur de 5 à 30% p/p_{association}.

Préférentiellement, l'huile de baobab est présente à une teneur de 40% p/p_{association} ; l'huile d'Abyssinie est présente à une teneur de 50% p/p_{association} ; l'huile de lin et/ou l'huile de carthame est présente à une teneur de 10% p/p_{association}.

Préférentiellement, l'huile de baobab est présente à une teneur de 40% p/p_{association} ; l'huile d'Abyssinie est présente à une teneur de 40% p/p_{association} ; l'huile de lin et/ou l'huile de carthame est présente à une teneur de 20% p/p_{association}.

Selon un autre aspect, l'invention propose une composition anhydre de soins pour cheveux texturés, notamment pour les cheveux afro-texturés. Cette composition anhydre est remarquable en ce qu'elle contient au moins 50% p/pcomposition anhydre, de préférence au moins 75% p/p_{composition} anhydre, préférentiellement au moins 95% p/p_{composition anhydre} de l'association d'huiles végétales selon la présente invention.

Selon un autre aspect, l'invention propose un procédé de soin des cheveux texturés, notamment des cheveux afro-texturés, comprenant l'application, sur les cheveux, de ladite association d'huiles végétales ou de ladite composition anhydre, en vue d'améliorer l'hydratation, la définition des boucles, la douceur, la brillance et/ou la facilité de démêlage desdits cheveux.

Le procédé de soin selon l'invention peut en outre comprendre une étape d'exposition des cheveux, après application de ladite association ou de ladite composition anhydre, à l'oxygène de l'air, à un rayonnement ultraviolet et/ou à une source de chaleur, ladite chaleur étant de préférence appliquée à une température comprise entre 25 °C et 100 °C.

De manière générale, l'application de ladite association ou de ladite composition anhydre conduit à la formation, à la surface des cheveux, d'un film protecteur résultant de la polymérisation de l'acide linoléique (C18:2, n-6) et/ou de l'acide linolénique (C18:3, n-3) présents dans ladite association.

Avantageusement, le film protecteur formé contribue à la rétention de l'humidité, à la protection des cheveux contre les agressions extérieures, notamment la chaleur, la pollution, les rayonnements UV et les produits chimiques, ainsi qu'à l'amélioration de la définition des boucles, de la douceur et de la brillance des cheveux.

En particulier, la chaleur est appliquée à l'aide d'un dispositif chauffant choisi parmi : un sèche-cheveux, un fer à lisser, un séchoir, une lampe chauffante, une baguette chauffante ou un casque chauffant.

### Brève description des figures.

D'autres avantages et caractéristiques de l'invention apparaîtront plus clairement à la lecture de la description des modes de réalisation préféré qui va suivre, en référence au dessin annexé, réalisé à titre d'exemples indicatif et non limitatifs et sur lesquels :

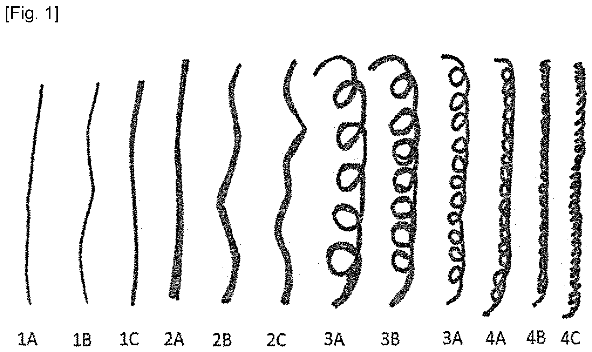

[Fig. 1] est une illustration de la classification d'André Walker des boucles de cheveux, qui comprend quatre types principaux : lisses, ondulés, bouclés et crépus.

### Description détaillée de l'invention.

La présente description est donnée à titre non limitatif, chaque caractéristique exposée seulement dans un mode de réalisation pouvant être généralisée aux autres modes de réalisation. De même, une ou plusieurs caractéristiques exposées seulement dans un mode de réalisation peuvent être combinées avec une ou plusieurs autres caractéristiques exposées seulement dans un autre mode de réalisation.

Dans toute la description, y compris les revendications, les proportions sont en poids, sauf si le contraire est spécifié.

Les expressions « compris entre ... et ... », « compris de ... à ... » et « allant de ... à ... » doivent se comprendre bornes incluses, sauf si le contraire est spécifié.

L'expression « constituée de » s'entend ici dans son acception fermée, c'est-à-dire que l'association d'huiles végétales est limitée exclusivement aux huiles explicitement mentionnées, à l'exclusion de tout autre ingrédient, qu'il soit actif ou auxiliaire. Autrement dit, la formulation ne contient aucun autre constituant que ceux spécifiés, et la somme des pourcentages indiqués représente la totalité (100 %) de l'association.

Par l'expression « traitement des cheveux», on entend désigner un traitement cosmétique non thérapeutique, permettant de résoudre les problèmes spécifiques des cheveux texturés, tels que la sécheresse, la rugosité et les frisottis, en améliorant leur peignage et leur démêlage, leur douceur, leur soyeux et leur brillance.

L'expression « p/p_{association} » signifie que le pourcentage du composant ou ingrédient considéré est en poids par rapport au poids total de l'association d'huiles végétales selon l'invention.

L'expression « p/p_{composition anhydre} » signifie que le pourcentage du composant ou ingrédient considéré est en poids par rapport au poids total de la composition de soins selon l'invention, se présentant sous forme anhydre.

L'expression « p/p_{composition aqueuse} » signifie que le pourcentage du composant ou ingrédient considéré est en poids par rapport au poids total de la composition de soins selon l'invention, se présentant sous forme aqueuse.

La présente invention repose sur la découverte qu'une association d'huiles végétales spécifique comprenant une huile de baobab, une huile d'Abyssinie, et une huile de lin et/ou une huile de carthame, s'avère efficace pour améliorer l'hydratation des cheveux texturés. En plus d'hydrater, cette association d'huiles offre une nutrition complète, une protection et un renforcement adaptés à ce type de cheveux. Elle est également efficace pour faciliter le peignage et le démêlage, améliorer la définition des boucles, ainsi que le toucher et la brillance des cheveux texturés. Ses composants sont rapidement absorbés par les cheveux sans laisser de film gras. En outre, une telle association d'huiles végétales est économique à produire, facilement reproductible et peut être produite à grande échelle.

Par « cheveux texturés » on désigne les cheveux de type 3 ou 4 selon la classification d'André Walker.

### Classification d'André Walker :

La classification d'André Walker est un système de classification des cheveux texturés qui a été conçu par un célèbre coiffeur dénommé André Walker. Cette classification (voir figure 1) est basée sur deux facteurs principaux : la texture du cheveu et le rebond du cheveu.

La texture du cheveu est classée sur une échelle de 1 à 4, de lisse à crépu :
- Type 1 : Cheveux lisses. - Type 2 : Cheveux ondulés.
- Type 3 : Cheveux bouclés à frisés.
- Type 4 : Cheveux très frisés à crépus (ou dit-cheveux afro-texturés).

En ce qui concerne le rebond du cheveu, cette classification distingue également trois sous-catégories, notées A, B et C, qui décrivent davantage les caractéristiques des boucles ou des ondulations.

En croisant ces deux facteurs, on obtient une classification de 12 catégories de cheveux, chacune ayant ses caractéristiques distinctes.
- Cheveux lisses (1A, 1B, 1C) ;
- Cheveux ondulés (2A, 2B, 2C) ;
- Cheveux bouclés (3A, 3B, 3C) ;
- Cheveux crépus (4A, 4B, 4C).

Cette classification est utile pour comprendre les besoins spécifiques des différents types de cheveux. Par exemple, les cheveux texturés de type 3 ou 4 , en fonction du climat, auront besoin d'être plus hydratés et protégés, car ils seront plus sujets à la sécheresse capillaire, aux agressions extérieurs et donc plus fragiles , dans ce contexte, que d'autres types de cheveux

### Association d'huiles végétales :

S'agissant de l'association d'huiles végétales spécifique, celle-ci comprend :
- une huile d'Abyssine (nom **INCI :** *Crambe Abyssinica Seed Oil*) *;*
   une huile de baobab (nom **INCI :** *Adansonia Digitata Seed Oil*) ; et
- une huile de lin (nom **INCI :** *Linum usitatissimum Seed oil*) et/ou une huile de carthame (nom **INCI :** *Carthamus tinctorius Seed Oil*)*.*

L'huile d'Abyssinie est présente dans l'association d'huiles végétales en une teneur d'au moins 20% p/p_{association}, de préférence en une teneur de 25 à 65% p/p_{association}.

L'huile d'Abyssinie est extraite des graines de Crambe Abyssinica. C'est une huile d'un jaune pâle à jaune doré. Elle peut être obtenue par toute méthode d'extraction connue de l'homme du métier, par exemple par pressage mécanique ou par extraction par solvant sous pression. Voir par exemple l'article de Francisco Aléxis Dntas Maia, et al.. Crambe Abyssinica Oil as Raw Material for Renewable Fuel Production- Review. Journal of Biotechnology and Biomedicine 6 (2023): 322-330. DOI:10.26502/jbb.2642-91280094. L'huile d'Abyssinie est également disponible dans le commerce.

L'huile de lin et/ou l'huile de carthame sont présentes dans l'association d'huiles végétales en une teneur d'au moins 5% p/p_{association}, de préférence en une teneur de 5 à 30% p/p_{association}.

L'huile de lin est extraite des graines de lin. Elle est reconnaissable à sa couleur jaune doré et son odeur légèrement prononcée. Elle peut être obtenue par toute méthode d'extraction connue de l'homme du métier, par exemple par pressage mécanique, par extraction par solvant organique (ex. n-hexane) sous pression, ou par extraction par fluide (ex. propane ou CO₂) subcritique. Voir par exemple le document de brevet CN106635389B ou l'article de Guilherme Sabadin Piva, et al., Linseed (Linum usitatissimum) Oil Extraction Using Different Solvents ; Food Technol Biotechnol. 2018 Sep; 56(3): 366-372. doi: 10.17113/ftb.56.03.18.5318. L'huile de lin est également disponible dans le commerce.

L'huile de Carthame est une huile extraite des graines de carthame (*Carthamus tinctorius L.*)*.* L'huile est principalement constituée de triglycérides d'acide linoléique. C'est une huile fluide de couleur jaune à l'odeur caractéristique. Elle peut être obtenue par toute méthode d'extraction connue de l'homme du métier, par exemple par pressage mécanique, par extraction par solvant organique (ex. n-hexane) sous pression, ou par extraction par fluide (ex. propane ou CO₂) subcritique. Voir par exemple le document de brevet CN101744237A ou l'article de : Nauman Khalid, et al.; "A comprehensive characterisation of safflower oil for its potential applications as a bioactive food ingredient - A review"; Trends in Food Science & Technology, Volume 66, 2017, Pages 176-186. https://doi.org/10.1016/j.tifs.2017.06.009. L'huile de carthame est également disponible dans le commerce.

L'huile de baobab est présente dans l'association d'huiles végétales en une teneur d'au moins 20% p/p*_{association}*, de préférence en une teneur de 25 à 65% p/p*_{association}.*

L'huile de baobab est extraite des graines du fruits du baobab (*Adansonia digitata L.*)*.* Elle est de couleur jaune à jaune foncé avec une odeur douce de noix. Elle peut être obtenue par toute méthode d'extraction habituelle connue de l'homme du métier, par exemple par pressage mécanique, par extraction par solvant organique (ex. n-hexane) sous pression, ou par extraction par fluide (ex. propane ou CO2) subcritique. Voir par exemple le document de brevet CN106635389B ou l'article de Hayford Ofori, et al. Impact of Different Oil Extraction Techniques on the Physicochemical Properties of Adansonia digitata Seed ; Int J Food Sci. 2023; 2023: 6233461 ; doi: 10.1155/2023/6233461. L'huile de baobab est également disponible dans le commerce.

Les huiles d'Abyssinie, de baobab, et de lin et/ou de carthame peuvent être employées, dans le cadre de la présente invention, comme huiles raffinées ou non-raffinées.

Le terme "huile raffinée" fait référence à une huile végétale qui a subi un processus de raffinage pour éliminer les impuretés, améliorer sa stabilité et modifier ses caractéristiques. Les processus de raffinage comprennent typiquement les étapes suivantes : Déterrage (les impuretés, telles que les particules solides, les pigments et les molécules odorantes, sont éliminées de l'huile) ; Blanchiment (l'huile est blanchie pour lui donner une couleur plus claire) ; Désodorisation (l'huile est désodorisée pour lui enlever son odeur naturelle) ; Neutralisation (l'acidité de l'huile est neutralisée).

Le terme "huile non-raffinée" fait référence à une huile végétale qui n'a pas été soumise à un processus de raffinage. Elle conserve donc toutes ses propriétés naturelles, y compris ses triglycérides et ses insaponifiables, notamment ses composés phytochimiques tels que des vitamines (par exemple la vitamine E), des stérols, des alcools gras, des hydrocarbures tels que des squalènes et des caroténoïdes. Bien que la quantité d'insaponifiables varie et dépende du type d'huile végétale, elle se situe généralement dans la plage de 0,5 à 5 % en poids par rapport au poids de l'huile végétale considérée. De tels composés phytochimiques peuvent contribuer aux effets bénéfiques des triglycérides (ou des acides gras essentiels).

De préférence, les huiles composant l'association de la présente invention, sont des huiles non raffinées, obtenues à partir des graines correspondantes par pressage mécanique suivie d'une filtration sur papier pour éliminer les particules solides susceptibles de rester en suspension dans l'huile correspondante.

Dans des modes de réalisation préférés, l'huile de baobab est présente dans l'association d'huiles végétales en une teneur de 30 à 60% p/p_{association} ; l'huile d'Abyssinie est présente dans l'association d'huiles végétales en une teneur de 30 à 60% p/p_{association} ; l'huile de lin et/ou l'huile de carthame est présente dans l'association d'huiles végétales en une teneur de 5 à 30% p/p_{association}.

Selon un mode particulier et préféré de l'invention, l'huile de baobab est présente dans l'association d'huiles végétales en une teneur de 40% p/p_{association} ; l'huile d'Abyssinie est présente dans l'association d'huiles végétales en une teneur de 50% p/p_{association} ; l'huile de lin et/ou l'huile de carthame est présente dans l'association d'huiles végétales en une teneur de 10% p/p_{association}.

Selon un autre mode particulier et préféré de l'invention, l'huile de baobab est présente dans l'association d'huiles végétales en une teneur de 40% p/p_{association} ; l'huile d'Abyssinie est présente dans l'association d'huiles végétales en une teneur de 40% p/p_{association} ; l'huile de lin et/ou l'huile de carthame est présente dans l'association d'huiles végétales en une teneur de 20% p/p_{association}.

Selon encore un autre mode particulier et préféré de l'invention, l'huile de baobab est présente dans l'association d'huiles végétales en une teneur de 50% p/p_{association} ; l'huile d'Abyssinie est présente dans l'association d'huiles végétales en une teneur de 40% p/p_{association} ; l'huile de lin est présente dans l'association d'huiles végétales en une teneur de 5% p/p_{association} ; et l'huile de carthame est présente dans l'association d'huiles végétales en une teneur de 5% p/p_{association}.

Les proportions respectives des trois ou quatre huiles composant l'association d'huiles végétales de l'invention peuvent être choisies de manière à fournir un apport équilibré en acides gras, en particulier en acides gras insaturés, afin de répondre aux besoins spécifiques des cheveux texturés, c'est-à-dire de leur offrir une hydratation en profondeur, une définition améliorée des boucles, une protection contre les dommages, une réduction de la sécheresse, une facilité accrue de peignage et de démêlage, ainsi qu'une brillance naturelle.

L'huile de Baobab, l'huile d'Abyssinie et l'huile de lin et/ou l'huile de carthame, apportent chacune à l'association d'huiles végétales selon l'invention divers acides gras, en particulier des acides gras instaurés, parmi lesquels on trouve l'acide oléique (C18:1, n-9), l'acide linoléique (C18:2, n-6), l'acide linolénique (C18:3, n-3), l'acide érucique (C22:1, n-9).

La [Table 1] ci-dessous montre les acides gras représentatifs des triglycérides et leur distribution dans les trois huiles végétales de l'association selon l'invention.

**[Table 1]**

| Acides gras | Huile de Baobab | Huile de lin | Huile de Carthame | Huile d'Abyssinie |
|---|---|---|---|---|
| <C16 | < 1% | < 1% | < 1% | < 1% |
| Acide palmitique (C16:0) | 15 à 30% | 3 à 8 % | 3 à 10 % | 1 à 8% |
| Acide stéarique (C18:0) | < 10% | < 10% | 1 à 5% | 1 à 5% |
| Acide oléique (C18:1, n-9) | 20 à 45% | 11 à 35 % | 7 à 20 % | 8 à 30% |
| Acide linoléique (C18:2, n-6) | 10 à 40% | 11 à 24 % | 65 à 85% | 3 à 17% |
| Acide linolénique (C18:3, n-3) | < 2% | 35 à 65 % | ≤ 1 % | 2 à 10% |
| Acide arachidique (C20) | < 1% | < 1% | ≤ 1 % | < 1% |
| Acide érucique (C22:1, n-9) | < 0,1% | < 0,1% | ≤ 0,5 % | 40 à 60% |

Les valeurs indiquées dans [Table 1] sont toutes données en % en poids sur la base de la quantité totale d'acides gras des triglycérides présents dans l'huile correspondante. Elles sont typiquement déterminées par analyse par chromatographie en phase gazeuse (GC). Ces valeurs ne sont que des valeurs approximatives et peuvent varier du fait que les huiles sont d'origine naturelle.

Grâce à la synergie des acides gras issus de l'huile de Baobab, de l'huile d'Abyssinie et de l'huile de lin, l'association selon la présente invention offre des avantages remarquables pour le soin des cheveux, en particulier pour les cheveux texturés, qui nécessitent une hydratation et une protection renforcées.

Tout d'abord, les acides linoléique (C18:2, n-6) et linolénique (C18:3, n-3) contenus dans l'association selon l'invention sont des acides gras polyinsaturés comportant des doubles liaisons réactives. Ces insaturations leur confèrent la capacité de polymériser et réticuler à la surface des cheveux. Plus précisément, ces acides gras polyinsaturés sont susceptibles de subir une polymérisation oxydative en présence de dioxygène (O₂), notamment lorsqu'ils sont exposés à l'air, à un rayonnement ultraviolet et/ou à la chaleur, par exemple à une température comprise entre 25 °C et 120 °C. De manière générale, lors de l'application de l'association d'huiles sur les cheveux, ce phénomène de polymérisation conduit à la formation d'un film protecteur à la surface des cheveux. Ce film présente plusieurs avantages : il améliore la rétention de l'humidité, contribuant ainsi à prévenir la sécheresse capillaire ; il agit également comme une barrière contre les agressions extérieures, telles que la chaleur, la pollution, les rayonnements UV ou les agents chimiques. En outre, il contribue à améliorer la douceur et la brillance des cheveux, ainsi qu'à optimiser la définition des boucles, ce qui est particulièrement bénéfique pour les cheveux texturés.

En outre, grâce à sa richesse en acides gras essentiels, notamment l'acide palmitique, l'acide oléique, l'acide linoléique, l'acide linolénique et l'acide érucique, l'association selon l'invention favorise la souplesse, la douceur et l'éclat des cheveux texturés. Ces cheveux deviennent plus faciles à coiffer et à démêler, limitant ainsi les risques de casse durant le brossage ou le coiffage.

De plus, les acides gras insaturés apportés par l'huile de Baobab, l'huile d'Abyssinie et l'huile de lin et/ou l'huile de carthame, contribuent également à renforcer la structure des cheveux, favorisant ainsi une croissance capillaire saine.

D'autre part, l'association selon l'invention se distingue par ses propriétés siccatives, définies comme la capacité d'un corps gras à évoluer, lorsqu'il est appliqué en couche mince, d'un état liquide à un état solide ou semi-solide (non fluide) sous l'effet de l'exposition à l'air, à la chaleur et/ou à la lumière. Ces propriétés siccatives permettent à l'association d'être rapidement absorbée par la fibre capillaire, en particulier dans le cas des cheveux texturés, sans laisser de film gras ni alourdir la chevelure. Cette absorption rapide améliore la sensation de légèreté après application et facilite la diffusion homogène des huiles sur la surface capillaire, ce qui contribue à l'efficacité globale du soin.

Enfin, un autre aspect important de l'association selon l'invention est sa capacité à nourrir le cuir chevelu, favorisant ainsi la santé capillaire globale. Possédant des propriétés apaisantes, régénérantes, cicatrisantes, antioxydantes et anti-inflammatoires, cette association peut également aider à traiter les problèmes du cuir chevelu, tels que la sécheresse, les démangeaisons et les pellicules.

### Compositions et procédés cosmétiques de soins capillaires

L'association d'huiles végétales de l'invention est, de préférence, mise en œuvre dans une composition cosmétique destinée aux soins capillaires, et avantageusement aux soins de cheveux texturés.

Cette composition cosmétique peut être une composition anhydre ou une composition aqueuse.

Par « anhydre », on entend une composition présentant une teneur en eau inférieure à environ 5 % en poids, en particulier inférieure à 2 % en poids, de préférence inférieure à 1 % en poids, voire inférieure à 0,5 % en poids, par rapport au poids total de la composition. De préférence, la composition cosmétique anhydre ne contient pas d'eau ajoutée.

Par « aqueuse », on entend une composition dont la teneur en eau libre est supérieure à 5% en poids, de préférence supérieure à 25% en poids, mieux supérieure à 50% en poids, voire supérieure à 70% en poids, par rapport au poids total de la composition.

### Composition anhydre :

S'agissant de la composition anhydre, l'association d'huiles végétales de l'invention y est avantageusement présente en une proportion d'au moins 50% p/pcomposition anhydre, de préférence d'au moins 75% préférentiellement d'au moins 95% p/p_{composition anhydre}.

La composition anhydre selon l'invention peut en outre contenir un ou plusieurs composants additionnels tels que les additifs habituellement utilisés, dans des compositions cosmétiques du type huiles sèches ou sérums non-aqueux. Les composants additionnels peuvent notamment être choisis parmi des antioxydants (ex : α-tocophérol, palmitate d' α-tocophérol, acétate d' α-tocophérol), des agents hydratants (ex : la glycérine, le propylène glycol et le butylène glycol), des agents parfumants (ex. les composants odorants tels que la vaniline, le nérol, le géraniol, le citronellol, le linalol, le cis-jasmone, le décanal, le citral, le salicylate d'amyle, l'alcool phényléthylique , la (-)-(R)-muscone et similaires ; et les huiles essentielles telles que l'huile de lavande, l'huile de citron, l'huile de citron vert, l'huile de mandarine, l'huile de marjolaine, l'huile de menthe poivrée, l'huile de menthe verte, l'huile de rose, l'huile de cèdre, l'huile de marjolaine, l'huile de myrrhe, l'huile de néroli, l'huile de patchouli, l'huile d'ylang ylang, et similaires), des huiles végétales distinctes des huiles de l'association selon la présente invention (par exemple : huile de ricin, huile d'amande douce, huile de coco, huile d'argan, huile de macadamia, huile de Moringa, huile d'arachide, huile de Canola, huile d'avocat, et similaires), des conservateurs (par exemple : alcool benzylique, phénoxyéthanol), et leurs mélanges. Les antioxydants peuvent aider à améliorer la stabilité au stockage de la composition anhydre. Les agents hydratants peuvent contribuer à maintenir l'hydratation des cheveux en attirant et/ou en retenant l'eau. Les autres huiles végétales éventuellement ajoutées à la composition anhydre peuvent fournir une variété d'acides gras essentiels, contribuant ainsi à améliorer la nutrition des cheveux, renforcer leur structure capillaire et préserver leur santé globale. Les agents parfumants (ou fragrances) peuvent jouer un rôle essentiel en masquant d'éventuelles odeurs désagréables provenant d'autres ingrédients de la composition anhydre. De plus, ils peuvent également aider à enrichir l'expérience sensorielle de l'utilisateur en créant des arômes agréables qui laissent une sensation de fraîcheur et de bien-être après utilisation. Les conservateurs peuvent aider à prévenir la croissance de de micro-organismes nuisibles (ex. bactéries, moisissures) dans la composition, ce qui maintient la stabilité microbiologique du produit et évite tout risque de contamination qui pourrait compromettre la sécurité et la qualité du produit cosmétique.

Bien entendu, l'homme du métier saura adapter le choix des éventuels composants additionnels à ajouter à la composition anhydre de l'invention et également le mode opératoire de telle manière que les propriétés avantageuses attachées intrinsèquement à la présente invention ne soient pas ou substantiellement pas altérées par l'addition envisagée. Ces composants additionnels peuvent être présents dans la composition anhydre de 0.001 à 50% en poids par rapport au poids total de la composition anhydre. Dans des modes de réalisation, les éventuels agents antioxydants, les agents conservateurs et/ou agents parfumants ou fragrances, peuvent être ajoutés à la composition anhydre à des concentrations de 0,01 à 5% en poids par rapport au poids total de la composition anhydre. Dans des modes de réalisation, les éventuels agents hydratants et/ou huiles végétales distinctes des huiles de l'association conforme à la présente invention, peuvent être inclus à des concentrations de 0,1 % à 50 % en poids par rapport au poids total de la composition anhydre.

Typiquement, la composition anhydre selon l'invention se présente sous forme d'huile ou de sérum non-aqueux.

### Compositions aqueuses :

S'agissant de la composition aqueuse, l'association d'huiles végétales de l'invention y est présente, de préférence, dans une proportion comprise entre 0,1 et 40% p/p_{composition aqueuse}, notamment entre 0,1 et 25% p/p_{composition aqueuse}, préférentiellement entre 1 et 15% p/p_{composition aqueuse}.

Cette composition aqueuse peut, outre de l'eau, également contenir un ou plusieurs solvants organiques, notamment hydrophiles, tels que les alcools en C1-C4, comme l'éthanol, l'isopropanol, et le n-butanol ; les polyols en C3-C7 comme le propylèneglycol, l'hexylèneglycol ou le glycérol ; les éthers de polyols en C3-C7 comme le monométhyl éther de diéthylène glycol, le monoéthyl éther de diéthylèneglycol, le mono-n-butyl éther de diéthylène glycol ou le monométhyléther de propylèneglycol ; ainsi que les alcools aromatiques comme l'alcool benzylique ou le phénoxyéthanol. En général, le ou les solvants organiques peuvent être présents à raison de 0,1 à 80% en poids et de préférence de 1 à 50% en poids, par rapport au poids de la composition aqueuse.

La composition aqueuse selon l'invention peut comprendre en outre un ou plusieurs ingrédients facultatifs classiques bien connus dans la technique, tels que des épaississants; des émulsionnants ; des tensioactifs choisis parmi les tensioactifs anioniques, cationiques, non ioniques, amphotères et/ou zwittérioniques; des alcools gras en C10-C30 ; des agents hydratants ; des protéines ; des gélifiants ; des cires ; des gommes ; des huiles distinctes des huiles végétales de l'association selon la présente invention ; des antioxydants ; des agents de conditionnement ; des agents antistatiques ; des agents antipelliculaires ; des vitamines ; des filtres solaires ; des pigments minéraux ou organiques ; colorés ou non colorés ; des colorants ; des agents nacrants et/ou opacifiants ; des agents séquestrants ; des polymères filmogènes ; des agents parfumants ou fragrances ; des eaux florales ; des extraits de plantes (ex. extrait d'aloés Vera) ; des conservateurs ; des ajusteurs de pH , des agents tampon ; des hydrolysats de protéines ; des agents de contrôle de la viscosité ; des agents gonflants ou des propulseurs. Des exemples de tels ingrédients facultatifs sont notamment cités dans le CTFA Cosmetic Ingredient Handbook, dixième édition (publié en 2004 par la Cosmetic, Toiletry, and Fragrance Association, Inc., Washington, D.C.), Bien entendu, l'homme du métier veillera à choisir ce ou ces éventuels ingrédients facultatifs et/ou leur quantité de manière telle que les propriétés avantageuses (notamment stabilité, esthétique et/ou performances) de la composition aqueuse selon l'invention ne soient pas, ou substantiellement pas, altérées par l'adjonction envisagée. Typiquement, de tels ingrédients facultatifs peuvent être présents dans la composition aqueuse selon l'invention en une quantité allant de 0,01 à 30 % en poids par rapport au poids total de la composition aqueuse.

En principe, la composition aqueuse selon l'invention a un pH compatible avec les cheveux, le cuir chevelu, la peau du visage, et toute autre zone de peau. Ce pH est convenablement compris entre 2 et 8 et de préférence dans la plage de 2,5 à 6,5, plus préférablement de 3 à 5,5 et de préférence encore de 3,5 à 5. Il peut être ajusté à la valeur désirée au moyen d'ajusteurs de pH habituellement utilisés en cosmétique ou bien encore à l'aide de systèmes tampons classiques. Les ajusteurs de pH peuvent être des agents acidifiants ou des agents alcalinisants. Parmi les agents acidifiants, on peut citer, à titre d'exemple, les acides minéraux ou organiques comme l'acide chlorhydrique, l'acide orthophosphorique, les acides carboxyliques comme l'acide acétique, l'acide tartrique, l'acide citrique, l'acide lactique, l'acide succinique, l'acide glutarique, l'acide adipique, l'acide maléique, l'acide fumarique ou l'acide phtalique. Parmi les agents alcalinisants, on peut citer, à titre d'exemple, l'ammoniaque, les hydroxydes de sodium ou de potassium, les carbonates alcalins comme le carbonate de sodium ou de potassium, les alcanolamines telles que les mono-, di- et triéthanolamines ainsi que leurs dérivés. Parmi les systèmes tampons classiques, on peut citer à titre d'exemple le système tampon acide citrique/citrate ou acide citrique /hydroxyde de sodium.

La viscosité de la composition aqueuse selon l'invention est typiquement comprise entre 100 et environ 20000 mPa.s, notamment entre 500 à 15000 mPa.s, en particulier entre 1000 à 10000 mPa.s, telle que mesurée avec n'importe quel rhéomètre ou viscosimètre approprié à 25 °C (ex. au moyen d'un viscosimètre du type BROOKFIELD).

La composition aqueuse de l'invention peut se présenter sous toutes les formes galéniques, plus ou moins fluides, classiquement utilisées pour une application topique sur les cheveux. Elle peut notamment être formulée sous la forme de solutions du type lotion ou sérum aqueux ; sous forme de gels aqueux ou hydro-alcoolique; sous forme dispersée notamment sous forme d'une émulsion huile-dans-l'eau ou eau-dans-l'huile, de consistance liquide plus ou moins épaisse telle que des laits, des crèmes plus ou moins onctueuses ou des gels. On peut également envisager une composition aqueuse sous la forme d'une mousse ou encore sous forme aérosol comprenant également un agent propulseur (ex. : l'air, le gaz carbonique, l'azote, le diméthyléther, les hydrocarbures tels que le propane, l'isobutane) sous pression.

De manière préférentielle, la composition aqueuse se présente sous forme d'un produit capillaire (pour cheveux) du type à rincer tel qu'un shampooing ou un après-shampoing, ou du type sans rinçage tel qu'une crème, un masque, un sérum ou une lotion capillaire. Elle peut encore se présenter sous forme de teinture ou de mascara capillaire à appliquer au pinceau ou au peigne.

Les compositions, anhydres ou aqueuses, de soins pour cheveux texturés selon la présente invention peuvent être obtenues par n'importe quelle méthode de formulation connue dans le domaine cosmétique. Leurs ingrédients respectifs peuvent être mélangés dans l'ordre et dans des conditions facilement déterminées par l'homme du métier.

Les compositions, de soins selon l'invention (anhydres ou aqueuses), peuvent être conditionnées sous diverses formes, notamment dans des contenants (par exemple, un tube en métal ou en plastique). Un contenant peut éventuellement être munis d'une pompe. Il peut être pressé pour distribuer une quantité souhaitée de la composition de soin qui y est contenue. La composition peut être distribuée sous forme de spray, d'aérosol, de liquide, de lotion, de lait ou de crème.

L'association d'huiles végétales selon l'invention et les compositions de soins conformes à l'invention, qu'elles soient anhydres ou aqueuses, sont notamment destinées à être utilisées pour améliorer l'état des cheveux texturés et faciliter leurs soins quotidiens, en particulier pour apporter ou améliorer la facilité de démêlage et de peignage, la définition des boucles, le toucher et la brillance des cheveux texturés.

L'invention est également relative à un procédé de traitement cosmétique destiné à améliorer l'état des cheveux texturés, notamment des cheveux afro-texturés. Ce procédé comprend, de manière générale, l'application sur les cheveux de l'association d'huiles végétales telle que définie dans la présente description, ou de la composition de soins capillaires comprenant ladite association. L'application peut être réalisée selon les modalités classiques d'utilisation des compositions capillaires. Par exemple : application d'un produit capillaire du type à laisser sur les cheveux tel qu'une huile capillaire, un sérum capillaire ou un spray capillaire sur les cheveux texturés secs ou peu mouillés ; ou application d'un produit capillaire du type à rincer tel qu'un shampooing ou un après-shampoing sur cheveux texturés mouillés ; ou application d'un produit capillaire du type sans rinçage tel qu'une crème, un sérum aqueux ou une lotion capillaire sur cheveux texturés secs ou peu mouillés. Après l'application de la composition de soins, un rinçage des cheveux à l'eau peut être effectué, par exemple, dans le cas de l'utilisation d'une composition de soins se présentant sous forme d'un produit capillaire à rincer tel que shampoing ou après-shampooing. L'association d'huiles ou la composition peut être laissée en place pendant une durée variable selon la nature du produit, généralement comprise entre 5 minutes et 1 heure, voire plus de 1 heure.

Selon un mode de réalisation particulier, le procédé comprend en outre une étape d'exposition des cheveux, après application de l'association d'huiles ou de la composition la contenant, à l'oxygène de l'air, à une source de chaleur et/ou à un rayonnement ultraviolet. Cette exposition favorise la polymérisation des acides gras polyinsaturés contenus dans l'association, en particulier de l'acide linoléique (C18:2, n-6) et/ou de l'acide linolénique (C18:3, n-3), conduisant à la formation d'un film protecteur à la surface des cheveux.

La chaleur peut par exemple être appliquée à une température comprise entre 25°C et 100 °C, au moyen d'un dispositif chauffant tel qu'un sèche-cheveux, un fer à lisser, un séchoir, une lampe chauffante, une baguette chauffante ou un casque chauffant. La durée d'exposition peut varier en fonction du type de cheveu, de son taux d'humidité et de l'effet recherché. La durée d'exposition thermique est typiquement comprise entre 5 minutes à 1 heure, selon le type de cheveu, le niveau d'humidité et l'effet recherché.

Le film protecteur ainsi formé contribue à plusieurs bénéfices capillaires : il améliore la rétention d'humidité au sein de la fibre, protège les cheveux contre les agressions extérieures (chaleur, pollution, rayons UV, produits chimiques), et optimise la définition des boucles. Il améliore également la douceur, la souplesse et la brillance des cheveux texturés, tout en réduisant les frisottis et la casse.

Ce mode de traitement est particulièrement adapté aux routines capillaires dites « thermo-actives », faisant intervenir une source de chaleur contrôlée pour maximiser les effets du soin. Il peut être intégré dans une routine hebdomadaire ou bihebdomadaire, et être associé à d'autres soins capillaires tels que shampooing doux, masque hydratant ou crème coiffante.

Conformément à un autre mode de réalisation, l'activation de la polymérisation peut également être déclenchée ou accélérée par exposition au rayonnement ultraviolet. Ce rayonnement peut être fourni soit par la lumière solaire naturelle, dans le cas d'une application en extérieur ou sous exposition directe au soleil, soit par une source artificielle telle qu'une lampe UV utilisée en salon de coiffure, émettant dans des longueurs d'onde appropriées à la polymérisation. L'exposition aux UV permet ainsi de favoriser ou d'initier la formation du film protecteur à la surface des cheveux, en complément ou en substitution à la chaleur. La durée d'exposition aux UV peut varier de 5 minutes à 1 heure, en fonction de la source utilisée et de l'intensité du rayonnement.

Le procédé de traitement cosmétique selon l'invention peut être effectué quotidiennement (au moins une fois par jour) ou deux ou plusieurs fois par semaine, pendant au moins un mois.

Un avantage du procédé de traitement cosmétique selon l'invention est qu'il apporte ou améliore la facilité de démêlage et de peignage. Un autre avantage de ce procédé est qu'il rehausse l'aspect visuel et la santé apparente des cheveux texturés en les rendant plus doux et agréables au toucher, en leur en apportant une très forte brillance et en améliorant la définition des boucles. Un autre avantage de ce procédé est qu'il favorise une meilleure rétention d'eau dans les cheveux, essentielle pour maintenir une hydratation adéquate. Encore un autre avantage significatif de ce procédé est qu'il contribue à la formation d'une barrière de protection autour des cheveux, offrant ainsi une protection contre la perte d'eau, ainsi que contre les agressions environnementales et autres facteurs externes.

Il est également envisagé de combiner un traitement pour les cheveux texturés utilisant une composition de soins selon la présente description, qui se présente sous forme anhydre, avec un traitement pour les cheveux texturés employant une composition de soins selon la présente invention, qui se présente sous forme aqueuse. Cette combinaison de traitements vise à offrir un soin capillaire plus complet et personnalisé, en tirant parti des avantages spécifiques de chaque type de composition de soin, qu'elle soit anhydre ou aqueuse. Elle permet d'optimiser les bénéfices des deux compositions, assurant ainsi une approche intégrée et efficace pour le soin des cheveux texturés au quotidien.

Les compositions soins pour cheveux texturés selon la présente divulgation peuvent être incorporées dans un kit. Par exemple, un kit peut inclure au moins une composition de soins pour les cheveux selon la présente divulgation, qu'elle soit anhydre ou aqueuse, et une ou plusieurs compositions supplémentaires, par exemple, une solution lavante pour cheveux, composition de coloration, un shampoing, un après-shampoing, etc. Les différentes compositions sont contenues séparément dans les kits. Les kits peuvent également comprendre des instructions et/ou explications pour utiliser les compositions de soins de l'invention incluses dans les kits ainsi que l'utilisation de toutes les autres compositions éventuellement incluses dans les kits.

L'association d'huiles végétales de l'invention et les compositions de soins la contenant sont faciles à appliquer et à utiliser. Elles présentent en outre l'avantage d'être remarquablement bien tolérées par les cheveux et le cuir chevelu. Elles ne provoquent aucune réaction d'intolérance ou d'irritation cutanée. Elles sont particulièrement importantes pour les cheveux texturés de type 3 (bouclés) et de type 4 (crépus), qui ont souvent tendance à être plus sujets à la sécheresse et à la cassure, et qui peuvent présenter des défis en termes de démêlage, de coiffage et de mise en forme selon le style souhaité. Cependant, elles peuvent également être bénéfiques pour les cheveux de type 1 (lisses) ou de type 2 (ondulés), particulièrement lorsqu'ils sont secs, abîmés ou affaiblis par des traitements chimiques, des produits cosmétiques inadaptés et/ou l'exposition au soleil, à l'eau de mer et/ou au à l'eau chlorée de piscines.

De manière avantageuse, les compositions, anhydre ou aqueuse, selon l'invention sont exemptes de silicones ou de composés à base de silicone, tels que les diméthicones, cyclométhicones ou autres dérivés siloxanés fréquemment utilisés dans les formulations capillaires. Cette absence de silicones présente plusieurs bénéfices pour les cheveux texturés. En particulier, elle permet d'éviter l'accumulation de résidus occlusifs sur la fibre capillaire, favorise une meilleure pénétration des actifs lipidiques issus des huiles végétales, et préserve la légèreté et la souplesse naturelle des cheveux, sans effet alourdissant ou filmogène artificiel.

Par ailleurs, l'absence de silicones facilite l'entretien du cuir chevelu et réduit le recours à des agents nettoyants agressifs lors du lavage, contribuant ainsi à maintenir l'équilibre hydrolipidique des cheveux texturés, qui sont particulièrement sensibles à la sécheresse et à la casse.

Les exemples suivants sont donnés à titre d'illustration de l'invention et n'ont pas de caractère limitatif.

### Exemples

### Exemple 1 : Associations d'huiles végétales et évaluation sur cheveux texturés.

Par les techniques usuelles, on a préparé différentes associations huileuses A.1 à A.8 dont les compositions sont indiquées dans [Table 2] ci-dessous (les pourcentages sont en poids par rapport au poids de l'association considérée).

**[Table 2].**

| Ingrédients | A.1 | A.2 | A.3 | A.4 | A.5 | A.6 | A.7 | A.8 |
|---|---|---|---|---|---|---|---|---|
| Huile d'Abyssinie* | 50% | 50% | - | 70% | 60% | 50% | 40% | 25% |
| Huile de lin* | - | 50% | 50% | 5% | 10% | 10% | 20% | 20% |
| Huile de baobab* | 50% | - | 50% | 25% | 30% | 40% | 40% | 55% |
| ** Disponibles auprès du distributeur H&B OILS CENTER Co. (Israël)* | | | | | | | | |

### Evaluation sur cheveux texturés :

Les associations huileuses A.1 à A.8 décrites dans l'exemple 2 ont été utilisées dans un test coiffeur réalisé avec un coiffeur professionnel.

Vingt volontaires de sexe féminin (âge ≥ 18 ans) ont été recrutées pour ce test coiffeur. Les cheveux des participantes étaient de texture de type 3 à 4 selon la classification d'André Walker. Entre J-7 et J-0, les volontaires ne devaient pas s'appliquer de produit de soin capillaire à base d'huile(s) végétale(s). A J-0, les volontaires se rendent chez le coiffeur sans s'être lavées les cheveux ni le jour ni la veille du rendez-vous.

Les participantes ont été réparties en quatre groupes de cinq. Chaque groupe a utilisé deux associations huileuses différentes.

Le coiffeur professionnel a sélectionné dix mèches de la zone frontale de chaque participante pour le test. Ces dix mèches ont été séparées en deux groupes de cinq. Pour chaque participante, cinq mèches ont été traitées avec une des associations huileuses (A.1, A.2, A.3 ou A.7), tandis que les cinq autres mèches ont reçu une association huileuse différente (A.4, A.5, A.6 ou A.8), conformément à ce qui est indiqué dans [Table 3] ci-dessous.

**[Table 3].**

| Groupes | Associations huileuses testées |
|---|---|
| Groupe 1 | A.1 |
| | A.4 |
| Groupe 2 | A.2 |
| | A.5 |
| Groupe 3 | A.3 |
| | A.6 |
| Groupe 4 | A.7 |
| | A.8 |

Des quantités égales des associations huileuses testées ont été appliquées sur les dix mèches sélectionnées de la zone frontale et la zone du dessus de la tête de chaque participante, sur des cheveux préférentiellement humidifiés à l'eau. La même quantité d'association huileuse a été appliquée sur chaque mèche, puis a été étalée sur les longueurs jusqu'aux pointes en frottant mèche par mèche afin de bien répartir le produit de manière uniforme sur chaque mèche, sans les démêler.

Les associations huileuses ont été évaluées sur les critères suivants : peignabilité, douceur, définition des boucles et brillance.

Ces critères ont été évalués par un jury composé d'une personne qui a noté chaque critère sur une échelle de 1 à 5 (1 = le critère n'est pas présent, 5= le critère est très présent) :
- Peignabilité : la peignabilité des cheveux a été évaluée par un test de peignage (facilité avec laquelle les cheveux peuvent être peignés et démêlés sans casser). Les cheveux sont classés de 1 (très difficile à peigner) à 5 (très facile à peigner).
- Effet soyeux : l'effet soyeux des cheveux a été évalué par un test de toucher. Les cheveux sont classés de 1 (très rugueux) à 5 (très doux).
- Définition des boucles : la définition des boucles des cheveux a été évaluée par une échelle visuelle. Les cheveux sont classés de 1 (boucles très diffuses) à 5 (boucles très définies).
- Brillance : la brillance des cheveux a été évaluée par une échelle visuelle. Les cheveux sont classés de 0 (très ternes) à 5 (très brillants).

La [Table 4] montre les scores obtenus pour chaque association huileuse testée.

**[Table 4].**

| Critères évalués | Scores obtenus | | | | | | | |
|---|---|---|---|---|---|---|---|---|
| | A.1 | A.2 | A.3 | A.4 | A.5 | A.6 | A.7 | A.8 |
| Peignabilité | 2 | 2 | 2 | 3 | 4 | 5 | 5 | 3 |
| Effet soyeux | 3 | 2 | 2 | 4 | 5 | 5 | 5 | 3 |
| Définition des boucles | 2 | 1 | 1 | 3 | 4 | 5 | 5 | 4 |
| Brillance | 1 | 2 | 2 | 3 | 5 | 5 | 5 | 3 |

Les résultats présentés dans [Table 4] montrent que les associations A.4 à A.8 de l'invention ont un effet très significatif et important sur la facilité de peignage et de démêlage des cheveux, ainsi que sur la définition des boucles, la brillance et l'effet soyeux.

### Exemple 2 : Sérum non-aqueux pour cheveux texturés

Par les techniques usuelles, on prépare une composition de soins pour cheveux texturés se présentant sous forme de sérum non-aqueux dont les ingrédients et les proportions sont indiqués [Table 4] ci-dessous. Les pourcentages sont en poids par rapport au poids de la composition de soins.

**[Table 4].**

| Ingrédients | Proportions (en %) | | | |
|---|---|---|---|---|
| Huile d'Abyssinie | 49,5 | 40 | 49,5 | 50 |
| Huile de lin | 10 | 5 | - | 5 |
| Huile de carthame | - | 5 | 10 | 5 |
| Huile de baobab | 40 | 50 | 40 | 39,7 |
| Antioxydant | 0,3 | - | 0,3 | 0,3 |
| Agent parfumant | 0,2 | - | 0,2 | - |

Ce sérum, appliqué une fois par jour sur les cheveux de type 3 ou 4, permet d'améliorer de façon appréciable l'aspect et l'éclat des cheveux. Ils deviennent plus faciles à peigner et à démêler, plus doux au toucher, moins rugueux et moins secs. En outre, ce sérum est rapidement absorbé par les cheveux. Il ne laisse pas de sensation de gras sur les mains lorsqu'on passe les mains dans les cheveux.

### Exemple 3 : Sérum aqueux pour cheveux texturés

Par les techniques usuelles, on prépare une composition de soins pour cheveux texturés se présentant sous forme de sérum aqueux. Cette composition de soins (sérum aqueux ) contient 5% en poids de l'association huileuse A.7 décrite dans l'exemple 1 et comprend en outre les ingrédients suivants : Acétate de tocophérol, Agent parfumant (Fragrance), Camphre, Eau (Aqua), Glycérine, Menthol, Peg-6 Caprylic/Capric Glycérides, Polysorbate-20.

On a appliqué ce sérum aqueux sur les cheveux texturés. On a observé d'excellentes performances en termes de démêlage, de peignage, de définition des boucles, d'effet soyeux et de forte brillance.

### Exemple 4 : Shampoing pour cheveux texturés

Par les techniques usuelles, on prépare des compositions de soins pour cheveux texturés se présentant sous forme de shampoings et comprenant, dans de l'eau déminéralisée, de 1 à 15% de l'association huileuse A.7 décrite dans l'exemple 1 et d'autres ingrédients incluant : Acide citrique (ajusteur de pH) ; Agents parfumants (Citral, Limonène, Linalol, et mélange d'huiles essentielles) ; Bétaïne (agent hydratant) ; Caprylyl/caprylique glucoside (tensioactif non ionique) ; Chlorure de sodium ; Disodium cocoyl glutamate (agent tensioactif) ; Glycérine (agent hydratant) ; Laurate de glucoside (tensioactif non ionique) ; Protéine de blé hydrolysée (agent Antistatique et Conditionneur capillaire) ; Sodium cocoyl glutamate (tensioactif anionique) ; Sodium cocoyl glutamate (tensioactif anionique) ; Sorbate de potassium (conservateur) ; Tocophérol (antioxydant).

## Revendications

1. Association d'huiles végétales, destinée aux soins des cheveux texturés, notamment des cheveux afro-texturés, ladite association étant constituée d'au moins 20% p/p_{association} d'huile d'Abyssine, d'au moins 20% p/p_{association} d'huile de baobab, et d'au moins 5% p/p_{association} d'huile de lin et/ou d'huile de carthame.

2. Association d'huiles végétales selon la revendication 1, **caractérisée en ce qu'**elle est capable de former un film protecteur à la surface des cheveux par polymérisation de l'acide linoléique (C18:2, n-6) et/ou de l'acide linolénique (C18:3, n-3) qu'elle contient, lorsqu'elle est appliquée sur les cheveux.

3. Association d'huiles végétales selon la revendication 2, **caractérisée en ce que** la polymérisation de l'acide linoléique (C18:2, n-6) et/ou de l'acide linolénique (C18:3, n-3) est activée par l'exposition à l'oxygène de l'air ambiant, à un rayonnement ultraviolet et/ou à la chaleur, notamment à une température comprise entre 25°C et 100°C.

4. Association d'huiles végétales selon l'une des revendications 1 à 3, **caractérisée en ce que** l'huile d'Abyssinie est présente en une teneur de 25 à 65% p/p_{association}.

5. Association d'huiles végétales selon l'une des revendications 1 à 4, **caractérisée en ce que** l'huile de lin et/ou l'huile de carthame sont présentes en une teneur de 5 à 30% p/p_{association}.

6. Association d'huiles végétales selon l'une des revendications 1 à 5, **caractérisée en ce que** la teneur en l'huile de baobab est de 25 à 65% p/p_{association}.

7. Association d'huiles végétales selon l'une des revendications 1 à 6, **caractérisée en ce que** l'huile de baobab est présente en une teneur de 30 à 60% p/p_{association} ; l'huile d'Abyssinie est présente en une teneur de 30 à 60% p/p_{association} ; et l'huile de lin et/ou l'huile de carthame sont présente en une teneur de 5 à 30% p/p_{association}.

8. Association d'huiles végétales selon l'une des revendications 1 à 7, **caractérisée en ce que** l'huile de baobab est présente en une teneur de 40% p/p_{association} ; l'huile d'Abyssinie est présente en une teneur de 50% p/p_{association} ; et l'huile de lin et/ou l'huile de carthame sont présentes en une teneur de 10% p/p_{association}.

9. Association d'huiles végétales selon l'une des revendications 1 à 8, **caractérisée en ce que** l'huile de baobab est présente en une teneur de 40% p/p_{association} ; l'huile d'Abyssinie est présente en une teneur de 40% p/p_{association} ; l'huile de lin et/ou l'huile de carthame sont présentes en une teneur de 20% p/p_{association}.

10. Composition anhydre de soins pour cheveux texturés, notamment pour les cheveux afro-texturés, **caractérisée en ce qu'**elle contient au moins 50% p/p_{composition} anhydre, de préférence au moins 75% p/p_{composition} anhydre, préférentiellement au moins 95% p/p_{composition anhydre} de l'association d'huiles végétales selon l'une des revendications 1 à 9.

11. Procédé de soin des cheveux texturés, notamment des cheveux afro-texturés, comprenant l'application, sur les cheveux, de l'association d'huiles végétales selon l'une des revendications 1 à 9 ou de la composition anhydre selon la revendication 10, afin d'apporter ou d'améliorer la facilité de démêlage et de peignage, la définition des boucles, le toucher et la brillance desdits cheveux texturés.

12. Procédé selon la revendication 11, comprenant en outre une étape d'exposition des cheveux, après application de ladite association ou de ladite composition anhydre, à l'oxygène de l'air, à un rayonnement ultraviolet et/ou à une source de chaleur, ladite chaleur étant de préférence appliquée à une température comprise entre 25 °C et 100 °C.

13. Procédé selon l'une des revendications 11 ou 12, dans lequel l'application de ladite association ou de ladite composition anhydre conduit à la formation, à la surface des cheveux, d'un film protecteur résultant de la polymérisation de l'acide linoléique (C18:2, n-6) et/ou de l'acide linolénique (C18:3, n-3) présents dans ladite association ou ladite composition.

14. Procédé selon la revendication 13, dans lequel le film protecteur formé contribue à la rétention de l'humidité, à la protection des cheveux contre les agressions extérieures, notamment la chaleur, la pollution, les rayonnements UV et les produits chimiques, ainsi qu'à l'amélioration de la définition des boucles, de la douceur et de la brillance des cheveux.

15. Procédé selon l'une des revendications 12 à 14, dans lequel la chaleur est appliquée à l'aide d'un dispositif chauffant choisi parmi : un sèche-cheveux, un fer à lisser, un séchoir, une lampe chauffante, une baguette chauffante ou un casque chauffant.
